# EUROPÄISCHE PATENTSCHRIFT

(11) **EP 1 112 722 B1**
(45) Veröffentlichungstag und Bekanntmachung des Hinweises auf die Patenterteilung: **23.03.2005**
(21) Anmeldenummer: 00122816.2
(22) Anmeldetag: 20.10.2000
(51) Int. Cl.: A61C 8/00

(54) **Implantat-Element mit selbstschneidendem Gewinde**
Self-tapping implant
Implant auto-taraudant

(30) Priorität: 29.12.1999 CH 239799
(43) Veröffentlichungstag der Anmeldung: 04.07.2001
(73) Patentinhaber: Hermann, Werner, CH-6312 Steinhausen (CH)
(72) Erfinder: Hermann, Werner, CH-6312 Steinhausen (CH)
(74) Vertreter: OK pat AG

(56) Entgegenhaltungen:
- FR-A- 2 737 847
- US-A- 4 668 191
- US-A- 5 061 181

## Beschreibung

Gegenstand der Erfindung ist ein Implantat-Element mit einem selbstschneidenden Rundgewinde nach dem Oberbegriff des Anspruchs 1.

Implantate dienen zur Richtung oder Verstärkung von beeinträchtigten Knochenbereichen oder als Ersatz für abgenutzte Knochenbereiche. Implantate zum Richten und Verstärken werden häufig zur Korrektur deformierter Wirbelsäulen benutzt; sie weisen mehrere Implantat-Elemente auf, nämlich Haltekörper, Schrauben bzw. Implantat-Elemente mit selbstschneidenden Gewinden sowie eine durchgehende Fixierstange. Die Haltekörper werden im wesentlichen übereinander an Wirbelkörpem befestigt, und zwar mit Hilfe der Implantat-Elemente, die ein selbstschneidendes Gewinde aufweisen; die Fixierstange wird von den Haltekörpern aufgenommen und ist beim stehenden Patienten im wesentlichen senkrecht angeordnet. Häufig dienen Implantate auch als Ersatz für abgenutzte Gelenke. Solche Gelenkimplantate bestehen aus zwei gelenkig miteinander verbundenen Implantat-Elementen, von denen das eine Implantat-Element an einem ersten Knochenrest und das andere Implantat-Element an einem zweiten Knochenrest befestigt wird; zu diesem Zwecke können die Implantat-Elemente selbstschneidende Gewinde aufweisen, um in den Knochenresten verschraubt zu werden. Andere Implantate wie zum Beispiel Kieferimplantate umfassen nur ein einziges Implantat-Element, auf welchem dann Zahnersatzteile befestigt werden, die aus dem Kieferknochen und aus dem Zahnfleisch ragen; auch die dafür verwendeten Implantat-Elemente besitzen selbstschneidende Gewinde.

Funktionell nehmen Gewinde von Implantat-Elementen, also eigentlich Knochengewinde, eine Mittelstellung zwischen Holzgewinden und Metallgewinden bzw. zwischen kraftschlüssigen und formschlüssigen Befestigungsbolzen, ein. Einerseits findet, wie bei Holzschrauben, durch das eingeschraubte Gewindeteil eine gewisse Verdrängung der spongiösen Knochensubstanz statt, was zur Folge hat, dass ein permanenter Druck auf das Implantat-Element und natürlich auch auf den umgebenden Knochenbereich entsteht. Anderseits wird, wie für Metallschrauben, im Knochen ein Muttergewinde geschnitten.

Herkömmliche Gewinde, mit welchen Implantat-Elemente üblicherweise versehen sind, weisen Gewindegänge auf, die - in einem die Gewindelängsachse enthaltenden Schnitt - durch Geraden begrenzt sind; Gewinde mit dreieckigem Querschnitt der Gewindegänge werden als Spitzgewinde und Gewinde mit trapezförmigem oder sägezahnartigem Querschnitt als Trapezgewinde bzw. Sägezahngewinde bezeichnet. Gewinde mit derartigen Querschnitten weisen zahlreiche Nachteile auf, von denen im Folgenden die Wichtigsten dargelegt werden.

Ein eingeschraubte Implantat-Element mit herkömmlichem Spitz-, Trapez- oder Sägezahngewinde ist wegen der Formgebung des Gewindes, das heisst wegen der scharfen Kanten im Kopf- und Kernbereich des Gewindes, unvorteilhaft, weil durch diese Kanten das Einwachsen des Implantat-Elementes im Knochen - genauer gesagt das Umwachsen des lmplantat-Elementes mit Knochensubstanz - im Bereich des Gewindes nachteilig beeinflusst wird. Erwiesenermassen fördern scharfe Kanten die Entstehung von Bindegewebe; dieses nimmt den Platz ein, an dem sich sonst Knochen bilden könnte, und verhindert dadurch eine solide Verankerung des Implantat-Elementes.

Die erwähnten scharfen Kanten haben weiterhin zur Folge, dass im Knochen unerwünschte Kerbwirkungen entstehen. Diese gehen aus vom Rillengrund der schraubenlinienförmigen Knochenrille, welche das selbstschneidende Gewinde während des Einschraubens im Knochen erzeugt. Nur nebenbei sei bemerkt, dass natürlich auch am Gewinde selbst Kerbwirkungen entstehen, die sich - vor allem weil das Gewinde auf Wechsellast beansprucht ist - negativ auf die Lebensdauer des Implantat-Elementes auswirken können.

Schliesslich sind die scharfen vorspringenden Kanten im Bereich des Gewindekopfes auch deshalb unvorteilhaft, weil die Gefahr besteht, dass sich Abriebpartikel aus dem Implantat-Werkstoff bilden.

Insbesondere bei Spitzgewinden, je nach Formgebung aber auch bei Trapez- bzw. Sägezahngewinden treten noch weitere Nachteile auf. Mindestens im Bereich des Gewinde-Kemdurchmessers - bei Gewinden mit sehr steilen Flanken aber auch zwischen zwei benachbarten Gewindegängen - wird durch die Flanken benachbarter Gewindegänge ein recht begrenztes Volumen bzw. ein sehr enger Kanal gebildet. Dies wirkt sich ungünstig auf die Bildung von Knochensubstanz aus. Knochensubstanz ist bekanntlich nicht ein isotropes bzw. kontinuierliches Material sondern weist eine Art dreidimensionaler Netz- bzw. Gitterstruktur auf. Aus diesem Grunde kann Knochensubstanz vom Körper nicht innerhalb eines beliebig kleinen Raumes gebildet bzw. regeneriert werden, sondern nur dann, wenn ein gewisses Minimalvolumen, genauer gesagt in jeder der drei räumlichen Dimensionen, eine gewisse Minimalstrecke zur Verfügung steht. Im begrenzten Raum im Bereich des Gewinde-Kerndurchmessers bzw. im engen Kanal zwischen zwei benachbarten Gewindeflanken kann daher praktisch kein Knochen sondern nur Bindegewebe entstehen, welches, wie schon wähnt, infolge seiner lockeren Natur dem Implantat-Element keine genügende Verankerungsmöglichkeit bietet.

Ein weiteres Problem bei der Verwendung von Implantat-Elementen mit herkömmlichen Spitz- und Trapezgewinden steht ebenfalls im Zusammenhang mit der Bildung bzw. Regeneration von Knochensubstanz. Es ist bekannt, dass sowohl unter einem zu geringen wie auch einem zu hohen Druck die Konservierung, Regeneration und Bildung von Knochensubstanz verhindert oder mindestens stark reduziert wird. Am besten gedeiht Knochensubstanz bei einer geeigneten, mässigen aber nicht konstanten Druckbeanspruchung. Zwar ändern sich die Beanspruchung des Knochens und des Implantat-Elementes über die Zeit, es ist aber bei herkömmlichen Gewinden unmöglich, diese Beanspruchung im erwünschten Rahmen zu halten. Es ist zu unterscheiden zwischen der Einschraub-Phase, den Ruhephasen bei eingeschraubtem Implantat-Element und den Benutzungsphasen bei eingeschraubtem Implantat-Element. Während der Einschraub-Phase stehen grundsätzlich die entgegengesetzt zur Einschraub-Richtung gewandten Flanken der Gewindegänge unter Druck, während die in Einschraubrichtung gewandten Flanken nicht belastet sind. Während der Benutzungs-Phasen wirkt die Druck-Belastung auf die in Einschraub-Richtung gewandten Flanken, während die entgegengesetzt zur Einschraub-Richtung gewandten Flanken nicht belastet sind. Während der Ruhe-Phasen verschwindet die Druck-Belastung oder ist allseitig sehr viel geringer als die maximalen Druck-Beanspruchung in der Einschraub-Phase oder in den Belastungs-Phasen. Bei der Beanspruchung von Implantat-Elementen mit herkömmlichen Gewinden, sowohl während der Einschraub-Phasen als auch während der Belastungs-Phasen, besteht nun die Gefahr, dass die jeweils nicht unter Druck-Beanspruchung stehenden Flanken in einen sogenannten Lastschatten geraten bzw. von jeder Druck-Belastung befreit sind oder ggfs. sogar unter einer negativen Druck-Belastung stehen. Auf die jeweils angrenzenden Knochenbereiche wirkt grundsätzlich eine gleich grosse entgegengesetzte Belastung, und entsprechend besteht auch die gleiche Gefahr des Entstehens von Lastschatten. Das heisst, dass nicht nur während der Ruhephasen der Druck auf den Knochen verschwindet, sondern dass auch während der Einschraub-Phase und vor allem während der Benutzungsphasen kein Druck auf den Knochen ausgeübt wird oder sogar ein Lastschatten auftritt. Diese Gefahr des Entstehens von Lastschatten ist bei den herkömmlichen Spitz- und Trapezgewinden gravierend; die Lastschatten treten nämlich hier während der Einschraub-Phase und der Benutzungsphasen jeweils nicht nur punktuell sondern längs der gesamten Flanke auf, da sich infolge der gerade verlaufenden Flanken ein praktisch konstanter Belastungsverlauf über die Flanke einstellt. Der negative Einfluss des Lastschattens auf die Konservierung, Regeneration und Bildung von Knochensubstanz und auf das Einwachsen der Implantat-Elemente wirkt sich daher gewissermassen flächendeckend auf die entsprechenden Flanken aus, wobei flache Flankenwinkel die Gefahr der Lastschattenbildung steigern.

Da die herkömmlicherweise an Implantat-Elementen verwendeten Spitz- und Trapezgewinde nicht befriedigen, wird versucht, Implantat-Elernente mit Rundgewinden zu verwenden. Schraubenschäfte solcher Implantat-Elemente bestehen im Wesentlich aus einem axial langen Schaftbereich, der von einer zylindermantelförmigen Hüllkurve begrenzt ist. An diesen Schaftbereich schliesst sich meist ein axial sehr kurzer Spitzenbereich an, der beim Einschrauben vorläuft, und der von einer zum Ende zulaufenden Hüllkurve begrenzt ist. Rundgewinde, die - mit Ausnahme eines axial kurzen Spitzenbereiches - durch zylindermantelförmige Hüllflächen begrenzt sind, die sich in die Gewindelängsachse enthaltenden Schnitten als Hüllkürven darstellen. Hüllkurven begrenzt sind, sind bekannt, beispielsweise aus der **US-5,061,181,** der **US-4,668,191** und der **FR-2 737 847,** wobei jeweils die Kontur der Gewindegänge über die axiale Ausdehnmung des Rundgewindes gleich bleibt. Der Nachteil solcher zylindrischer Rundgewinde besteht darin, dass häufig durch die Keilwirkung eine Klemmen eintritt.

Es ist daher **Aufgabe** der Erfindung, ein Implantat-Element mit einem Rundgewinde zu schaffen, mit welchem die Nachteile des Standes der Technik vermieden oder mindestens stark reduziert werden können.

Die **Lösung** dieser Aufgabe erfolgt erfindungsgemäss durch die Merkmale des kennzeichnenden Teils des Anspruchs **1**.

Vorteilhafte Weiterbildungen des erfindungsgemässen Implantat-Elementes sind durch die abhängigen Ansprüche **2** bis **9** definiert.

Das Implantat-Element nach der Erfindung weist somit ein Gewinde auf, das als Rundgewinde ausgebildet und das durch eine zulaufende Hüllkurve begrenzt ist. Jeder der Gewindegänge umfasst wie üblich einen vorspringenden Bereich und einen der angrenzenden rückspringenden Bereiche. Beim neuen Rundgewinde nimmt die axiale Ausdehnung der vorspringenden Bereiche zu je mehr die Hüllkurve zuläuft, das heisst je kleiner der Gewindedurchmesser wird.

Durch die Form des neuen Gewindes wird die Bildung von Bindegewebe praktisch unterbunden und die Bildung von Knochen dadurch gefördert; das Einwachsen des Gewindebereiches kann daher in optimaler Weise stattfinden, so dass das Implantat-Element bestens verankert wird.

Ausserdem werden die bei der Verwendung von Spitzgewinden auftretenden Kerbwirkungen im Knochen vermieden.

Vorteilhaft sind auch die auf der neuen Gewindeausbildung basierenden Effekte am Implantat-Element selbst; durch das Rundgewinde werden nicht nur Kerbwirkungen vermieden, sondern es vermindert sich auch die Gefahr von Abrieb von Implantat-Material im Bereich der spitzen, in den Knochen vorspringenden Gewindebereiche, welche sich für den Impantat-Träger schädlich auswirken könnten.

Im Weiteren ist das neue Rundgewinde so ausgebildet, dass die Konservierung bzw. Regenerierung bzw. Bildung von Knochen möglichst gefördert bzw. mindestens nicht allzustark behindert wird. Dies bedeutet, dass für die Gewinde-Dimensionierung bezüglich Flankenneigung und Steigung ein Kompromiss angestrebt werden muss. Einerseits müssen die Flankenneigung und die Gewindesteigung gross sein, damit ein in jeder Dimension genügend weiter Raum für die Konservierung, Regeneration und Bildung von Knochensubstanz entsteht; damit vermeidet man die Bildung von Bindegeweben anstelle von Knochensubstanz. Anderseits dürfen die Flanken nicht allzu steil verlaufen, damit die Gefahr von Lastschattenbildung möglichst vermieden wird.

Zahlenmässige Werte für die genaue Formgebung und Dimensionierung der Rundgewinde könnten nur in so weiten Grenzen erfolgen, dass sie keine sinnvollen Anhaltspunkte für die Herstellung der Gewinde liefern würden. Der Grund dafür liegt darin, dass Implantate bzw. deren Implantat-Elemente und demzufolge auch die entsprechenden Gewinde an unterschiedlichen Körperbereichen zur Verwendung kommen und daher auch sehr unterschiedlich ausgebildet sein können. Hierbei spielen nicht nur die relativen sondern auch die absoluten Dimensionen der Implantat-Elemente bzw. der Gewinde eine bestimmte Rolle. Im Weiteren müssen auch die Eigenschaften der Werkstoffe, aus dem die Implantat-Elemente hergestellt sind, berücksichtigt werden; hierbei bestehen insbesondere grosse Unterschiede zwischen herkömmlichen metallischen Werkstoffen wie Titan und in Zukunft vermutlich vermehrt zum Einsatz kommenden Kunststoffen.

Das neue Implantat-Element kann allein, beispielsweise im Dentalbereich zur Verankerung von Zahnersatz im Kieferknochen, verwendet werden. Es kann aber auch in Zusammenwirkung mit einem zweiten Implantat-Element als Implantat-Einheit verwendet werden; insbesondere eignet sich eine solche Implantat-Einheit zur Verankerung von künstlichen Hüftgelenken einerseits im Beckenknochen und anderseits im Oberschenkelknochen; hierbei ist es nicht zwingend, dass beide Implantat-Elemente der Implantat-Einheit Rundgewinde nach der Erfindung aufweisen.

Weitere Einzelheiten und Vorteile der Erfindung werden im Folgenden anhand von Ausführungsbeispielen des erfindungsgemässen Implantat-Elementes und mit Bezug auf die Zeichnung ausführlich erläutert. Es zeigen:
- **Fig. 1**: ein erstes Gewinde für ein Implantat-Element nach der Erfindung, in einem die Gewindelängsachse enthaltenden Schnitt;
- Fig. 2: ein zweites Gewinde für ein Implantat-Element nach der Erfindung, in gleicher Darstellung wie Fig. 1;
- **Fig. 3A**: ein Gewindegang eines weiteren Gewindes für ein Implantat-Element nach der Erfindung, mit der während der Einschraubphase wirkenden Druck-Beanspruchung, in einem die Gewindelängsachse enthaltenden Schnitt;
- **Fig. 3B**: den in Fig. 3A dargestellten Gewindegang, jedoch mit der in einer Ruhephase wirkenden Druck-Beanspruchung, in gleicher Darstellung wie Fig. 3A;
- **Fig. 3C**: den in Fig. 3A und Fig. 3B dargestellten Gewindegang, jedoch mit der in einer Belastungs-Phase wirkenden Druck-Beanspruchung, in gleicher Darstellung wie Fig. 3A und Fig. 3B;
- **Flg. 3D**: den zeitlichen Verlauf der Druck-Belastung auf drei Punkte einer Flanke des in den Fig. 3A bis 3C dargestellten Gewindeganges.
- **Fig. 4**: ein Implantat-Element nach der Erfindung, welche als Hüftgelenkpfanne bzw. zum Einschrauben in den Beckenknochen vorgesehen ist; und
- **Fig. 5**: ein Implantat-Element nach der Erfindung, welche als Basis für einen Zahnersatz bzw. zum Einschrauben in einen Kieferknochen vorgesehen ist

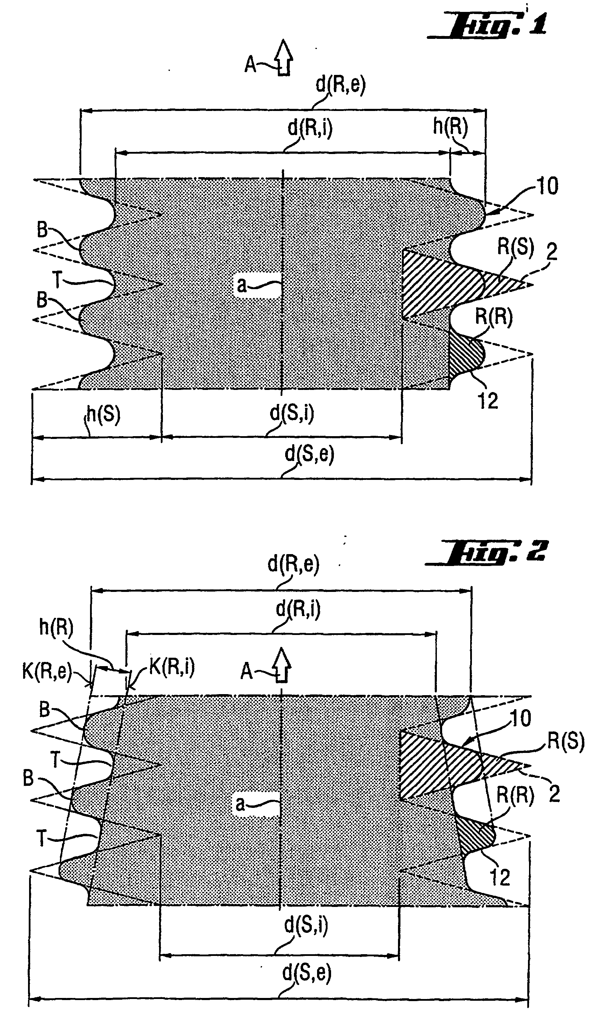

In der folgenden Beschreibung werden für entsprechende Teile der verschiedenen Ausführungsbeispiele stets dieselben Bezugszeichen verwendet, auch wenn die Teile nicht vollkommen gleich ausgebildet sind. Die Figuren sind nicht massstäblich, und insbesondere die **Fig. 2A, 2B** und **2C** sind lediglich als vereinfachte schematisierte Darstellungen zu betrachten.

In **Fig. 1** ist ein Teil eines Implantat-Elementes **10** dargestellt, welches mit einem Gewinde versehen ist. Das Gewinde ist entsprechend dem Stand der Technik ais zylindermantelförmig begrenztes als Rundgewinde **12** ausgebildet und mit einer durchgezogenen Linie dargestellt. Das Rundgewinde **12** besitzt eine Gewindetängsachse **a** und ist dazu bestimmt, in Richtung des Pfeiles **A** in einen nicht dargestellten Knochen eingeschraubt zu werden. Dank der grosszügigen Rundungen werden am Rundgewinde **12** alle scharfen Kanten vermieden, mit der Folge, dass bei seiner Verwendung das Entstehen von Bindegewebe und jede Kerbwirkung sowie die Gefahr des Entstehens von Abriebpartikeln vermieden wird.

Ebenfalls in **Fig. 1** ist, gewissermassen dem Gewinde **12** überlagert, ein herkömmliches zylindrisches Spitzgewinde **2** mit gestrichelter Linie dargestellt. Der Gewinde-Innendurchmesser bzw. -Kerndurchmesser **d(R, i)** des Rundgewindes **12** ist grösser als der Gewinde-Innendurchmesser **d(S, i)** des herkömmlichen Spitzgewindes **2**. Im Gegensatz dazu ist der Gewinde-Aussendurchmesser **d(R, e)** des Rundgewindes **12** kleiner als der Gewinde-Aussendurchmesser **d(S, e)** des Spitzgewindes **2.** Die Gewinde-Höhe **h(R)** des Rundgewindes **12** ist demzufolge beträchtlich kleiner als die Gewindehöhe **h(S)** des herkömmlichen Spitzgewindes **2**. Beim Einschrauben des Implantat-Elementes **10** entsteht im Knochen durch das selbstschneidende Rundgewinde **12** eine schraubenlinienförmige Knochenrille **R(R)**, die komplementär zum schraubenlinienförmigen Vorsprung des Rundgewindes **12** ausgebildet und in **Fig. 1** mit einer ersten, engen Schraffur dargestellt ist. Entsprechend würde im Knochen durch das selbstschneidende Spitzgewinde **2** eine schraubenlinienförmige Rille **R(S)** entstehen, die komplementär zum schraubenlinienförmigen Vorsprung des Spitzgewindes **2** ausgebildet wäre und in **Fig. 1** mit einer zweiten, weiten Schraffur versehen ist. Es ist deutlich zu erkennen, dass die durch das Rundgewinde **12** erzeugte Knochenrille **R(R)** im Querschnitt die Form eines **U** besitzt, während die durch das Spitzgewinde **2** erzeugte Knochenrille **R(S)** im Querschnitt die Form eines **V** besitzen würde. Die mittlere Flankenneigung ist beim Rundgewinde **12** gleich wie es die konstante Flankenneigung beim Spitzgewinde **2** wäre. Es ist ebenfalls leicht ersichtlich, dass die Knochenrille **R(R)** selbst an ihrem Rillengrund nie so eng ist wie es die Knochenrille **R(S)** des Spitzgewindes **12** an dessen Rillengrund wäre. Dadurch wird beim Rundgewinde **12** die Konservierung bzw. Regeneration oder Bildung von Knochensubstanz erleichtert.

**Fig. 2** zeigt ein weiteres Implant-Element **10** mit einem weiteren Rundgewinde **12**, das konisch ausgebildet ist. Der Gewinde-Innendurchmesser **d(R, i)** und der Gewinde-Aussendurchmesser **d(R, e)** des Rundgewindes **12** sind hier nicht konstant sondern ändern sich kontinuierlich längs des Rundgewindes **12** bzw. längs der Achse **a**. Auch bei diesem Ausführungsbeispiel werden scharfe Kanten vermieden und die durch das Rundgewinde **12** im Knochen erzeugte Rille **R(R)** ist breit. Die äussere Begrenzung des Rundgewindes bildet hier eine Konusfläche **K(R, e)** und die innere Begrenzung des Rundgewindes **12** bildet eine Konusfläche **K(R, i)**. Beim in **Fig. 2** dargestellten Ausführungsbeispiel besitzen die Konusflächen **K(R, e)** und **K(R, i)** die gleichen Neigungen zur Gewindelängsachse **a**.

Es sind auch andere Anordnungen denkbar; zum Beispiel können die Neigungen der beiden Konusflächen **K(R, e)** und **K(R, i)** ungleich sein; im weiteren kommen anstelle von zwei Konusflächen mit Geraden als Erzeugenden auch zwei andere zulaufende Flächen mit geeigneten parallelen oder nicht-parallelen Kurven als Erzeugenden in Frage.

Die Höhe **h(R)** des Vorsprungs des Rundgewindes **12** ist beim dargestellten Ausführungsbeispiel über seine gesamte axiale Länge bzw. für alle seine Gewindegänge konstant. Wie bei der Anordnung gemäss **Fig. 1**, bildet auch hier das Rundgewinde **12** einen Ausschnitt aus dem fiktiven Spitzgewinde **2**. Dadurch erhält man die im folgenden beschriebene, spezielle Gewinde-Konfiguration: Die axiale Ausdehnung der gesamten Gewindegänge des Rundgewindes **12**, bestehend aus je einem vorspringenden Bereich oder 'Berg' **B** sowie einem zwei vorspringende Bereiche trennenden 'Tal' **T** ist über die gesamte axiale Länge des Rundgewindes **12** konstant. Der über den Gewinde-Innendurchmesser **d(R, i)** vorstehende Vorsprung bzw. 'Berg' **B** wird jedoch mit abnehmendem Durchmessers **d(R, i)** breiter bzw. nimmt in axialer Richtung zu, während gleichzeitig mit abnehmendem Durchmesser **d(R, i)** die Rille bzw. das 'Tal' **T** schmaler wird bzw. in axialer Richtung abnimmt. Mit dieser speziellen Ausbildung erhält man ein Gewinde, das ein knochenschonendes Einschrauben sowie eine sehr gute Verankerung des Implantat-Elementes gewährleistet.

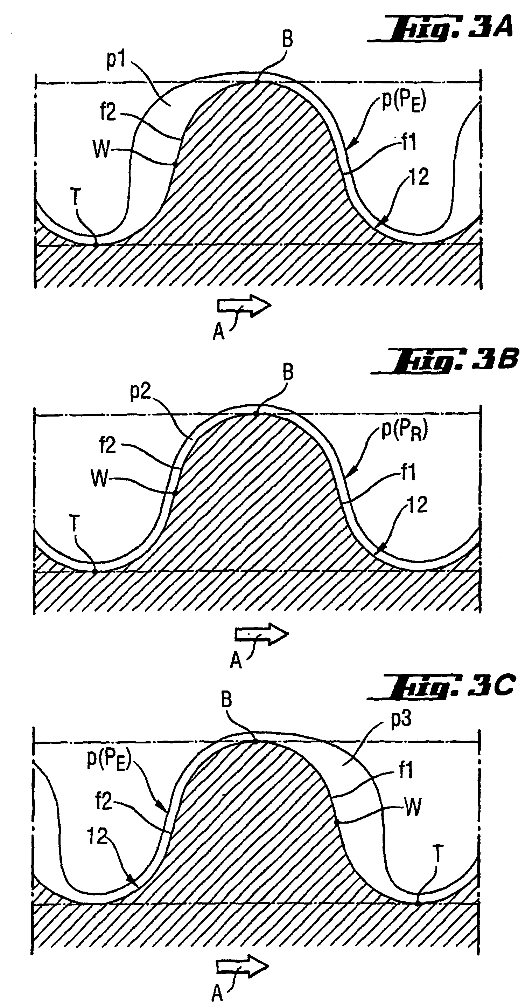

Die **Fig. 3A, 3B** und **3C** zeigen die Beanspruchung des Rundgewindes.

**Fig. 3A** zeigt für einen Gewindegang des Rundgewindes **12** den örtlichen Verlauf des Drukkes **p(P**_{**E**}**)** auf einen Gewindegang des Rundgewindes **12** während der Einschraub-Phase, wobei das Rundgewinde **12** in Richtung des Pfeiles **A** in einen nicht dargestellen Knochen bzw. Restknochen eingeschraubt wird. Die in Einschraubrichtung, das heisst in Richtung des Pfeiles **A** vorlaufende Flanke **f1** des Rundgewindes **12** unterliegt einem immer vorhandenen Basis-Druck. Auf die in Einschraubrichtung nachlaufende Gewindeflanke **f2** wirkt ein örtlich variabler Druck **p1**, der sich längs der Flanke **f2** des Rundgewindes **12** kontinuierlich ändert. Der im Längsschnitt durch das Rundgewinde **12** als Kopf bzw. 'Berg' **B** vorspringende Bereich und der als Rille bzw. 'Tal' **T** rückspringende Bereich unterliegen hierbei dem geringsten Druck, während der mittlere Bereich der Flanke **f2** - wo in der Kontur des Längsschnittes der Flanke **f2** ein Wendepunkt **W** vorhanden ist - am meisten belastet ist. Da beim Rundgewinde **12** der maximale Druck nicht im Bereich des Kopfes bzw. 'Berges' **B** auftritt, ist vorteilhafterweise die Deformationsgefahr geringer als bei einem Spitzgewinde. Dass die Druckbelastung bei **T** ebenfalls minimal ist, ist deshalb vorteilhaft, weil der Knochen, in welchen das Rundgewinde **12** eingeschraubt wird, jeweils durch den dem Druck **p** entgegengesetzten Reaktiv-Druck belastet ist; der maximale Reaktiv-Druck trifft den Knochen vorteilhafterweise nicht im Bereich **T** des Rundgewindes, wo vom Knochen nur noch ein axial schmaler Vorsprung übrig ist, sondern im Bereich **W** des Rundgewindes **12**, wo in axialer Richtung schon eine beträchtliche Knochenmasse und nicht nur ein axial schmaler Knochenrest vorhanden ist.

**Fig. 3B** zeigt den örtlichen Druckverlauf **p(P**_{**R**}**)** m eingeschraubten, jedoch nicht beanspruchten Rundgewinde **12,** also während einer Ruhephase **P**_{**R**}. Der im wesentlichen konstante Druck entspricht einem Basis-Druck **p2** und ist längs des gesamten Rundgewindes und insbesondere an den Flanken **f1** und **f2** konstant. Durch geeignete Dimensionierung der Gewindeparameter kann erreicht werden, dass der Basis-Druck **p2** in einem Bereich liegt, der für die Knochenbildung optimal ist. Diese uniforme Druckbelastung entspricht etwa der minimalen Druckbelastung bei **B** und bei **T** während der Einschraub-Phase **P**_{**E**} gemäss **Fig. 3A** und während der Beanspruchungsphase **P**_{**B**} gemäss **Fig. 3C**.

**Fig. 3C** zeigt den Druckverlauf **p(P**_{**B**}**)** beim eingeschraubten Rundgewinde **12** während der Beanspruchungsphase **P**_{**B**}**.** Dieser Druckverlauf **p(P**_{**B**}**)** ist praktisch spiegelsymmetrisch zur in **Fig. 3A** dargestellten Druckverlauf **p(P**_{**E**}**)** während der Einschraubphase **P**_{**E**}**,** das heisst, dass der Druck auf die Flanke **f2** sowie bei **B** und **T** dem Basis-Druck entspricht, während der Druck **p3** auf die Flanke **f1** bei **W** maximal ist. Hierbei ergeben sich dieselben Vorteile wie sie mit Bezug auf die Einschraubphase bzw. **Fig. 3A** erläutert wurden.

Unter der Voraussetzung einer geeigneten Dimensionierung existiert die Gefahr des Entstehens eines Lastschattens kaum. Selbst wenn während der Einschraubphase **P**_{**E**} oder während einer Benutzungsphase **P**_{**B**} ein Lastschatten auftreten würde, so würde sich dieser nicht an der gesamten Flanke **f1** bzw. **f2** sondern nur im Bereich des jeweiligen Wendepunktes **W** manifestieren; dies ist die Folge der nicht-konstanten Druckverteilung, welche ihrerseits die Folge der nicht-geradlinigen Form der Flanken **f1, f2** im Längsschnitt ist.

Das Rundgewinde **12** und natürlich auch der das Rundgewinde **12** umgebende Knochen unterliegen, wie dies aus den **Fig. 3A** bis **3C** hervorgeht, einer Wechsel-Last. Betrachtet man in den **Fig. 3A** bis **3C** jeweils einen Punkt des Rundgewindes **12** bzw. die dort auftretenden Druck-Belastungen **p1, p2, p3,** so erhält man den zeitlichen Verlauf dieser Wechsel-Last **p(f1),** der in **Fig. 3D** für die drei Punkte **B, W** und **T** der Flanke **f1** dargestellt ist. Zwar ist die Anzahl der Lastwechsel grundsätzlich längs des gesamten Rundgewindes **12** gleich, aber die Differenz zwischen den Extremalwerten des Druckes ist über die Flanken **f1, f2** unterschiedlich und hierbei vorteilhafterweise im Bereich **B** und im Bereich von **T** sehr gering. In den hervorspringenden und damit schwächsten Teilen des Rundgewindes **12** im Bereich **B** und natürlich auch im Bereich **T** sind somit die Unterschiede zwischen den Extremalwerten der Wechsellast nicht vorhanden oder unbedeutend. Entsprechend gibt es auch praktisch keine Unterschiede zwischen den

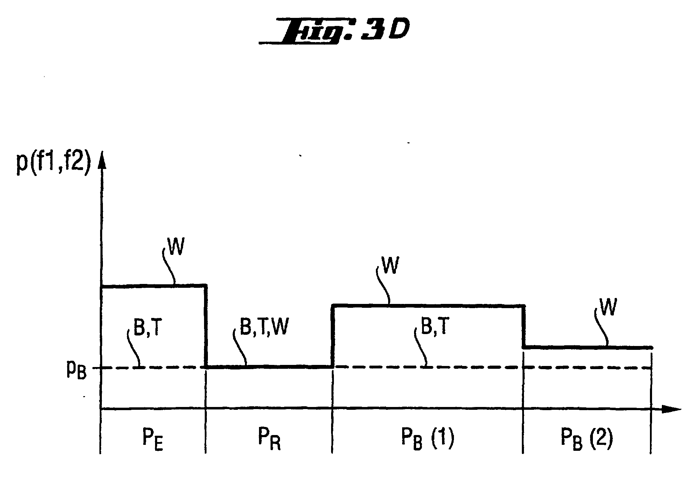

**Fig. 4** zeigt ein halbkugelähnliches Implantat-Element **10** mit einem zulaufenden Rundgewinde **12**, das nur wenige Gewindegänge aufweist. Dieses Implantat-Element **10** wird in Richtung des Pfeiles **A** so eingeschraubt, dass sein Pol-Bereich **90** vorläuft und sein Äquator-Bereich **0** nachläuft. Dieses Implantat-Element **10** ist dazu bestimmt, in einen Plattenknochen eingeschraubt zu werden, insbesondere in einen Beckenknochen, um die eine Hälfte eines Hüftgelenkimplantates zu bilden.

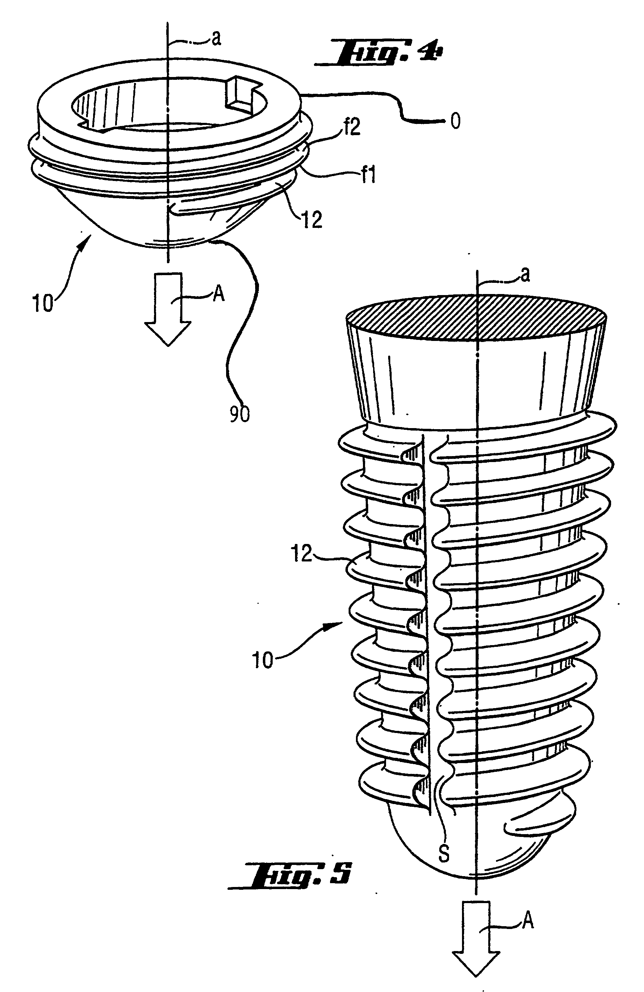

In **Fig. 5** ist ein längliches Implantat-Element **10** mit einem zulaufenden Rundgewinde **12** und zahlreichen Gewindegängen. Dieses Implantat-Element **10** kann beispielsweise im Dentalbereich Verwendung finden, eignet sich aber auch dazu, in einen Röhrenknochen eingeschraubt zu werden.

Es ist deutlich zu erkennen, dass die Hüllkurve, welche das Rundgewinde 12 begrenzt, über die gesamte axiale Länge des Gewindes konisch zulaufend ist, oder, mit anderen Worten, dass das Implantat-Element keinen oberen Schaftbereich aufweist, der durch eine zylindermantelförmige Hüllkurve begrenzt wäre.

Das Rundgewinde **12** des neuen Implantat-Elementes **10** ist ein selbstschneidendes Gewinde. Insbesondere in einer Realisation gemäss **Fig. 5** weist es mindestens eine Ausnehmung **S,** im allgemeinen aber mehrere solcher Ausnehmungen, auf, welche sich über die gesamte Gewindelänge erstrecken und die Funktion von Span-Nuten übernehmen, um beim Einschrauben vom Knochen abgetrennte Knochenpartikel wegzufördern. Diese Ausnehmungen **S** verlaufen mindestens annähernd parallel zur Gewindelängsachse **a**. Die durch die Ausnehmungen **S** gebildeten vorlaufenden Kanten des restlichen Gewindeprofils werden vorteilhaft als Schneidekanten ausgebildet. Das Vorhandensein dieser Ausnehmung **S** führt zu einer besonders guten Verankerung des Implantat-Elementes im Knochen, weil dadurch zusätzlich zum schraubenlinienförmigen Raum für Knochenmasse zwischen den Gewindegängen innerhalb der kanalartigen Ausnehmungen **S** weiterer Raum für Knochenmasse vorhanden ist.

## Patentansprüche

1. Implantat-Element **(10)** mit einem selbstschneidenden Rundgewinde **(12), das** mehrere Gewindegänge **(B, T)** mit vorspringenden Bereichen **(B)** aufweist,
**dadurch gekennzeichnet,**
- **dass** das Rundgewinde **(12)** durch eine distal zulaufende Hüllkurve begrenzt ist, und
- **dass** mit abnehmendem Gewindedurchmesser **(d(R,i), d(R,a))** die axiale Ausdehnung der vorspringenden Bereiche **(B)** der Gewinde-gänge **(B, T)** zunimmt.

2. Implantat-Element **(10)** nach Anspruch **1**,
**dadurch gekennzeichnet,**
**dass** die Hüllkurve mindestens annähernd hemisphärisch ist.

3. Implantat-Element **(10)** nach Anspruch **1,**
**dadurch gekennzeichnet,**
**dass** die Hüllkurve mindestens annähernd konisch ist.

4. Implantat-Element **(10)** nach Anspruch 3,
**dadurch gekennzeichnet,**
**dass** die Gewindegänge des Rundgewindes **(12)** Ausschnitte aus einem zylindrischen Spitzgewinde **(2)** sind, wobei der in Richtung der Gewindelängsachse **(a)** veränderliche Kemdurchmesser **(d(R,i))** des Rundgewindes **(12)** durch eine zulaufende Fläche **(Ki)** und der in Richtung der Gewindelängsachse **(a)** veränderliche Aussendurchmesser **(d(R,e))** des Rundgewindes **(12)** durch eine zulaufende Fläche **(Ke)** definiert ist.

5. Implantat-Einheit **(10)** nach Anspruch **4,**
**dadurch gekennzeichnet,**
**dass** die zulaufenden Flächen **(Ki, Ke)** parallel sind.

6. Implantat-Element **(10)** nach einem der Ansprüche **1** bis **5,**
**dadurch gekennzeichnet,**
**dass** die Gewindeflanken **(f1, f2)** des Rundgewindes **(12)** im Profil gekrümmt sind und höchstens im Bereich eines Wendepunktes **(W)** einen geraden Bereich aufweisen.

7. Implantat-Element **(10)** nach einem der Ansprüche **1** bis **6,**
**dadurch gekennzeichnet,**
**dass** die halben Flankenwinkel des Rundgewindes **(12)** im Bereich des Wendepunktes **(W)** mindestens annähernd im Bereich von 3° bis 60° liegen.

8. Implantat-Element **(10)** nach einem der Ansprüche **1** bis **7,**
**dadurch gekennzeichnet,**
**dass** die Rundungsradien im Bereich des Innendurchmessers **(d(R,i))** und des Aussendurchmessers **(d(R,e))** des Rundgewindes **(12)** mindestens annähernd im Bereich von 0.5 mm bis 10 mm liegen.

9. Implantat-Element **(10)** nach einem der Ansprüche 1 bis 8,
**dadurch gekennzeichnet,**
**dass** es mindestens eine längliche, mindestens annähernd parallel zur Gewindelängsachse **(a)** gerichtete, vorzugsweise bis auf den Kerndurchmesser **(d(R,i))** gehende Ausnehmung **(S)** aufweist, welche eine Span-Nute bildet, wobei vorzugsweise die vorlaufenden Kanten der restlichen Gewindegänge als Schneidkanten ausgebildet sind.

## Claims

1. Implant element **(10)** with a self-tapping round thread **(12)** comprising a plurality of turns of the thread **(B, T)** with projecting zones **(B),**
**characterized in that**
- the round thread **(12)** is delimited by a distally tapering envelope curve, and
- the axial length of the projecting zones **(B)** of the turns of the thread **(B, T)** increases with the decreasing thread diameter **(d(R, i), d(R, a)).**

2. Implant element **(10)** according to Claim **1,**
**characterized in that**
the envelope curve is at least approximately hemispherical.

3. Implant element **(10)** according to Claim **1**,
**characterized in that**
the envelope curve is at least approximately conical.

4. Implant element **(10)** according to Claim **3**,
**characterized in that**
the turns of the thread of the round thread **(12)** are sections of a cylindrical pointed thread **(2)** such that the core diameter **(d(R, i))** of the round thread **(12)**, which changes in the direction of the longitudinal axis **(a)** of the round thread **(12)** is defined by a tapering surface **(Ki)** and the outer thread diameter **(d(R, e))** of the round thread **(12)**, which changes in the direction of the longitudinal axis **(a)** of the round thread **(12),** is defined by a tapering surface **(Ke).**

5. Implant element **(10)** according to Claim **4,**
**characterized in that**
the tapering surfaces **(Ki, Ke)** are parallel.

6. Implant element **(10)** according to one of Claims **1** to **5**,
**characterized in that**
the flanks **(f1, f2)** of the round thread **(12)** seen in profile are bent and can only display a straight zone in the vicinity of a turning point **(W).**

7. Implant element **(10)** according to one of Claims **1** to **6,**
**characterized in that**
the half flank angles of the round thread **(12)** in the vicinity of the turning point **(W)** lie at least approximately in the range 3° to 60°.

8. Implant element **(10)** according to one of Claims **1** to **7,**
**characterized in that**
the radii of curvature in the vicinity of the inner diameter **(d(R, i))** and of the outer diameter **(d(R, e))** of the round thread **(12)** lie at least approximately in the range 0.5 mm to 10 mm.

9. Implant element **(10)** according to one of Claims **1** to **8,**
**characterized in that**
it possesses at least one longitudinal flute **(S),** preferably descending as far as the inner diameter **(d(R, i)),** at least approximately parallel to the axis **(a)** of the thread and constituting a chip groove, the leading edges of the part of the thread that remains after formation of the flutes preferably taking the form of cutting edges.

## Revendications

1. Élément d'implant (10) présentant un filet rond autotaraudeur (12) qui présente plusieurs pas de filetage (B, T) avec des zones saillantes (B),
**caractérisé en ce que**
- le filet rond (12) est délimité par une courbe enveloppe distale rentrante, et **en ce que**
- lorsque le diamètre de filet (d(R, i), d(R, a)) diminue, l'expansion axiale des zones saillantes (B) des pas de filet (B, T) augmente.

2. Élément d'implant (10) selon la revendication 1,
**caractérisé en ce que** la courbe enveloppe est au moins approximativement hémisphérique.

3. Élément d'implant (10) selon la revendication 1,
**caractérisé en ce que** la courbe enveloppe est au moins approximativement conique.

4. Élément d'implant (10) selon la revendication 3,
**caractérisé en ce que** les pas de filet du filet rond (12) sont des découpes d'un filet triangulaire cylindrique (2), le diamètre à fond de filet (d(R, i)) du filet rond (12) qui se modifie dans la direction de l'axe longitudinal de filet (a) étant défini par une surface qui se rétrécit (Ki) et le diamètre extérieur (d(R, e)) du filet rond (12) qui se modifie dans la direction de l'axe longitudinal de filet (a) étant défini par une surface qui se rétrécit (Ke).

5. Élément d'implant (10) selon la revendication 4,
**caractérisé en ce que** les surfaces rentrantes (Ki, Ke) sont parallèles.

6. Élément d'implant (10) selon l'une des revendications 1 à 5,
**caractérisé en ce que** les flancs de filet (f1, f2) du filet rond (12) ont un profil courbé et présentent au plus dans la zone d'un point d'inflexion (W) une zone rectiligne.

7. Élément d'implant (10) selon l'une des revendications 1 à 6,
**caractérisé en ce que** les demi-angles de flanc du filet rond (12) dans la zone du point d'inflexion (W) sont au moins approximativement dans la gamme de 3° à 60°.

8. Élément d'implant (10) selon l'une des revendications 1 à 7,
**caractérisé en ce que** les rayons d'arrondi dans la zone du diamètre intérieur (d(R, i)) et du diamètre extérieur (d(R, e)) du filet rond (12) sont au moins approximativement compris dans la gamme de 0,5 mm à 10 mm.

9. Élément d'implant (10) selon l'une des revendications 1 à 8,
**caractérisé en ce qu'**il présente au moins un évidement (S) longitudinal, orienté au moins approximativement parallèlement à l'axe longitudinal de filet (a), de préférence allant jusqu'au diamètre à fond de filet (d(R, i)), lequel forme une rainure de serrage, les arêtes avant des pas de filetage restants étant de préférence formées en tant qu'arêtes de coupe.
